Europäisches Patentamt

European Patent Office

Office européen des brevets

(11) Numéro de publication : **0 131 689**
**B1**

## (12)     FASCICULE DE BREVET EUROPÉEN

(45) Date de publication du fascicule du brevet :
11.01.89

(21) Numéro de dépôt : **84103550.4**

(22) Date de dépôt : **19.03.82**

(60) Numéro de publication de la demande initiale en
application de l'article 76 CBE : **0074984**

(51) Int. Cl.⁴ : **A 61 F 13/18, A 41 B 13/02**

(54) **Articles d'hygiène pour l'absorption des liquides corporels tels que des couches-culottes.**

(30) Priorité : 19.03.81 FR 8105477

(43) Date de publication de la demande :
23.01.85 Bulletin 85/04

(45) Mention de la délivrance du brevet :
11.01.89 Bulletin 89/02

(84) Etats contractants désignés :
AT BE CH DE GB LI LU NL SE

(56) Documents cités :
FR-A- 2 134 748
FR-A- 2 374 890
FR-A- 2 388 515
US-A- 3 814 100

(73) Titulaire : **KAYSERSBERG SA**
**Route de Lapoutroie**
**F-68240 Kaysersberg (FR)**

(72) Inventeur : **Pigneul, Raymond**
**2, rue des Vosges**
**F-68320 Durrennentzen (FR)**

(74) Mandataire : **David, Daniel**
**KAYSERSBERG 54, avenue Hoche**
**F-75008 Paris (FR)**

## Description

La présente demande résulte de la division de la demande européenne N° 82900961.2 (WO-A-82/03170) du 19.3.1982 déposée sous priorité française du 19.3.1981......

La présente invention se rapporte aux couches-culottes pour l'absorption des liquides corporels du type connu du FR-A-2388515. Elle est tout particulièrement adaptée à la réalisation d'une couche-culotte à jeter.

De tels articles présentent une structure stratifiée constituée d'un matelas d'absorption et de rétention des liquides (matelas de fibres cellulosiques) disposé entre une feuille perméable aux liquides en contact avec le corps humain, et une feuille extérieure imperméable aux liquides. A cette structure s'ajoutent des moyens pour assurer un bon maintien en position de l'article sur le corps.

La présente invention propose une nouvelle structure de couche-culotte selon la revendication 1.

Ceci permet d'éliminer de façon plus efficace les fuites latérales, de favoriser l'absorption et la rétention par l'effet de « poche » de la structure nouvelle.

D'autres avantages et caractéristiques de l'invention apparaîtront dans la description d'un exemple de réalisation, accompagné d'un dessin dans lequel :

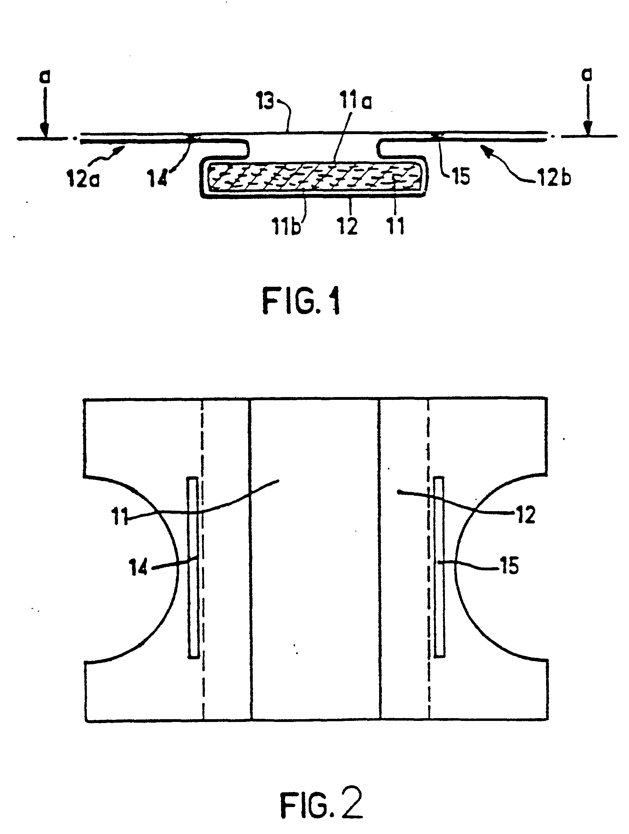

Les figures 1 et 2 représentent, respectivement en vue de dessus et en section droite, une couche-culotte à jeter conforme à la présente invention.

La couche-culotte est constituée d'une structure stratifiée comportant un matelas absorbant 11, une feuille extérieure imperméable aux liquides 12, et une feuille perméable aux liquides 13.

La feuille extérieure imperméable est repliée par « retour » sur les bords longitudinaux de la face 11a du matelas, dirigée vers le corps de l'utilisateur (bébé ou adulte incontinent).

D'autre part, des extensions latérales 12a, 12b, portent les éléments élastiquement extensibles 14 et 15, qui peuvent être par exemple, des laminettes de caoutchouc naturel ou synthétique posées à l'état tendu dans la région de l'entrejambe.

Une feuille perméable aux fluides, référencée 13, est fixée sur l'ensemble constitué par la masse absorbante et la feuille extérieure en forme de « poche ».

Une telle structure présente l'avantage de former une barrière contre les fuites latérales tout en maintenant la plus grande surface possible d'absorption en contact avec le corps.

## Revendications

1. Couche-culotte pour l'absorption des liquides corporels du type comprenant un matelas de matière absorbante (11) avec une face (11a) tournée vers le corps humain et une face (11b) opposée, revêtue d'une feuille extérieure (12) imperméable aux liquides qui enveloppe ledit matelas sur ladite face opposée (11b) sur toute l'épaisseur dudit matelas, puis, par retour, est appliquée sur les bords longitudinaux de la face du matelas (11a) dirigée vers le corps humain, puis est repliée sur elle-même au-dessus de chaque bord longitudinal de manière à présenter des extensions latérales (12a, 12b) sur lesquelles dans la zone d'entrejambe sont fixés, hors de la surface délimitée par le matelas absorbant (11), des éléments élastiquement extensibles (14, 15), caractérisés et en ce que l'ensemble formé par lesdites extensions latérales et le matelas est revêtu d'une feuille perméable aux liquides (13), fixée à la feuille imperméable aux liquides (12) sur les extensions latérales de cette dernière.

2. Couche-culotte à jeter, selon la revendication 1, caractérisée en ce que lesdits éléments élastiquement extensibles (14, 15) sont rectilignes et situés à une distance du matelas absorbant voisine de l'épaisseur dudit matelas.

## Claims

1. Nappy-pants for the absorption of body liquids of the type comprising a pad of absorbent matelas (11) with one face (11a) turned towards the human body and one opposed face (11b), covered with a liquid-impervious outer sheef (12) wich enveloppes the said pad on the said opposed face (11b) over the whole thickness of the said pad and then, in return, is applied onto the lengthwise edges of the face of the pad (11a), which is directed towards the human body, and is then folded back on itself above each lenghtwise edge so as to have side extensions (12a, 12b) onto which elastically extensible members (14, 15) are fastened in the crotch region, outside the area delimited by the absorbent pad (11), characterised in that the asembly formed by the said side extensions and the pad is covered with a liquid-permeable sheet (13), fastened to the liquid-impervious sheet (12) on the side extensions of the latter.

2. Disposable nappy-pants according to claim 1, characterised in that the said elastically extensible members (14, 15) are rectilinear and situated at a distance from the absorbent pad which is close to the thickness of the said pad.

## Patentansprüche

1. Höschenwindel zur Aufnahme von Körperflüssigkeiten mit einem aus flüssigkeitsabsorbierenden Material bestehenden Kissen (11) mit einer dem menschlichen Körper zugewandten Seite (11a) und einer abgewandten Seite (11b), das mit einer äußeren flüssigkeitsundurchlässigen Folie (12) umgeben ist, die das Kissen auf der abgewandten Seite (11b) in seiner gesamten Dicke umhüllt und weiterhin an den Längskanten der Seite (11a) des Kissens, die dem menschlichen Körper zugewandt ist, weiterhin oberhalb und entlang jeder Längskante gefaltet ist, so daß Längsansätze (12a, 12b) entstehen, auf denen in dem Zwischenbeinbereich außerhalb der Fläche, die durch das absorbierende Kissen (11) begrenzt wird, elastisch ausdehnbare Elemente (14, 15) vorgesehen sind, dadurch gekennzeichnet, daß die durch die Längsansätze und das Kissen gebildete Einheit durch eine flüssigkeitsdurchlässige Folie (13) bedeckt wird, die auf den seitlichen Ansätzen der flüssigkeitsundurchlässigen Folie befestigt ist.

2. Höschenwindel zum Wegwerfen nach Anspruch 1, dadurch gekennzeichnet, daß die elastischen ausdehnbaren Elemente (14, 15) geradlinig sind und in einer Entfernung von dem absorbierenden Kissen vorgesehen sind, die in etwa der Dicke des Kissens entspricht.

FIG. 1

FIG. 2